# EUROPEAN PATENT APPLICATION

(11) **EP 2 383 249 A1**
(43) Date of publication of application: **02.11.2011**
(21) Application number: 11164481.1
(22) Date of filing: 02.05.2011
(51) Int. Cl.: C07C 51/02, C07C 51/44, C07C 51/46

(54) **Processes for producing adipic acid from fermentation broths containing diammonium adipate**

(30) Priority: 30.04.2010 US 329800 P; 14.04.2011 US 87001
(71) Applicant: BioAmber S.A.S., Montreal H3B 4W8 (CA)
(72) Inventor: Fruchey, Olan S., Hurricane, WV 25526 (US); Keen, Brian T., Pinch, WV 25156 (US); Albin, Brooke A., Charleston, WV 25303 (US); Clinton, Nye A., Hurricane, WV 25526 (US); Dombek, Bernard D., Charleston, WV 25314 (US); Manzer, Leo E., Wilmington, DE 19803 (US); Dunuwila, Dilum, Princeton, NJ 08540 (US)
(74) Representative: Epping - Hermann - Fischer

(57) **Abstract**

Processes for making AA from either a clarified DAA-containing fermentation broth or a clarified MAA-containing fermentation broth that include distilling the broth under super atmospheric pressure at a temperature of >100°C to about 300°C to form an overhead that comprises water and ammonia, and a liquid bottoms that includes AA, and at least about 20 wt% water; cooling the bottoms to a temperature sufficient to cause the bottoms to separate into a liquid portion and a solid portion that is substantially pure AA; and separating the solid portion from the liquid portion. A method also reduces the broth distillation temperature and pressure by adding an ammonia separating and/or water azeotroping solvent to the broth.

## Description

### Related Application

This application claims the benefit of US Provisional Application No. 61/329,800, filed April 30, 2010, the subject matter of which is hereby incorporated by reference.

### Technical Field

This disclosure relates to processes for the direct production of adipic acid (AA) from fermentation broths containing diammonium adipate (DAA), monoammonium adipate (MAA), and/or AA.

### Background

Certain carbonaceous products of sugar fermentation are seen as replacements for petroleum-derived materials for use as feedstocks for the manufacture of carbon-containing chemicals. One such product is AA.

It would therefore be desirable to have a process for the direct production of substantially pure AA from a DAA-containing fermentation broth.

### Summary

We provide a process for producing AA from a clarified DAA-containing fermentation broth including distilling the broth under super atmospheric pressure at a temperature of >100°C to about 300°C to form an overhead that comprises water and ammonia, and a liquid bottoms that comprises AA and at least about 20 wt% water, cooling and/or evaporating the bottoms to attain a temperature and composition sufficient to cause the bottoms to separate into a liquid portion and a solid portion that is substantially pure AA, and separating the solid portion from the liquid portion.

We also provide a process for producing AA from a clarified DAA-containing fermentation broth including adding an ammonia separating solvent and/or a water azeotroping solvent to the broth, distilling the broth at a temperature and pressure sufficient to form an overhead that comprises water and ammonia, and a liquid bottoms that comprises AA and at least about 20 wt% water, cooling and/or evaporating the bottoms to attain a temperature and composition sufficient to cause the bottoms to separate into a liquid portion and a solid portion that is substantially pure AA, and separating the solid portion from the liquid portion.

We further provide a process for making AA from a clarified MAA-containing fermentation broth including distilling the broth under super atmospheric pressure at a temperature of >100°C to about 300°C to form an overhead that comprises water and ammonia, and a liquid bottoms that comprises AA and at least about 20 wt% water, cooling and/or evaporating the bottoms to attain a temperature and composition sufficient to cause the bottoms to separate into a liquid portion and a solid portion that is substantially pure AA, and separating the solid portion from the liquid portion.

We further provide a process for making AA from a clarified MAA-containing fermentation broth including adding an ammonia separating solvent and/or a water azeotroping solvent to the broth, distilling the broth at a temperature and pressure sufficient to form an overhead that includes water and ammonia, and a liquid bottoms that comprises AA, and at least about 20 wt% water, cooling and/or evaporating the bottoms to attain a temperature and composition sufficient to cause the bottoms to separate into a liquid portion and a solid portion that is substantially pure AA, and separating the solid portion from the liquid portion.

### Brief Description of the Drawings

Fig. 1 is a block diagram of a process for making AA from a DAA containing broth.

Fig. 2 is a graph showing the solubility of AA in water as a function of temperature.

### Detailed Description

It will be appreciated that at least a portion of the following description is intended to refer to representative examples of processes selected for illustration in the drawings and is not intended to define or limit the disclosure, other than in the appended claims.

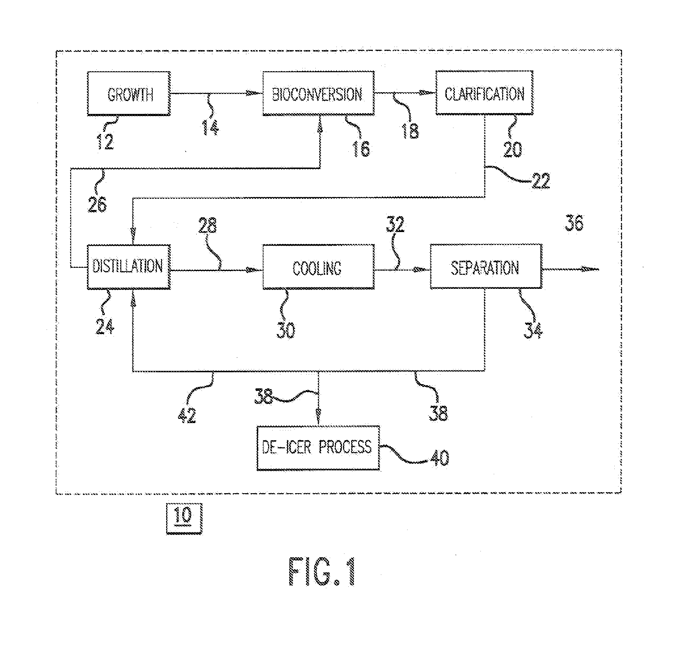

Our processes may be appreciated by reference to Fig. 1, which shows in block diagram form one representative example, 10, of our methods.

A growth vessel 12, typically an in-place steam sterilizable fermentor, may be used to grow a microbial culture (not shown) that is subsequently utilized for the production of the DAA, MAA and/or AA-containing fermentation broth. Such growth vessels are known in the art and are not further discussed.

The microbial culture may comprise microorganisms capable of producing AA from fermentable carbon sources such as carbohydrate sugars. Representative examples of microorganisms include *Escherichia coli (E. coli), Aspergillus niger, Corynebacterium glutamicum* (also called *Brevibacterium flavum*), *Enterococcus faecalis, Veillonella parvula, Actinobacillus succinogenes, Paecilomyces varioti, Saccharomyces cerevisiae, Candida tropicalis, Bacteroides fragilis, Bacteroides ruminicola, Bacteroides amylophilus, Lebsiella pneumonae* mixtures thereof and the like.

Preferred microorganisms include the *Candid tropicalis* (Castellani) Berkhout, anamorph strain OH23 having ATCC accession number 24887, *E. coli* strain AB2834/pKD136/pKD8.243A/pKD8.292 having ATCC accession number 69875, the *E. coli* cosmid clones designated 5B12, 5F5, 8F6 and 14D7 comprising a vector expressing the cyclohexanone monoxygenase having the amino acid sequence shown in SEQ ID NO: 2 and encoded by SEQ ID NO: 1 from *Acinetobacter* strain SE19, and the yeast strain available from Verdezyne, Inc. (Carslbad, CA, USA; hereinafter "Verdezyne Yeast") which produces AA from alkanes and other carbon sources.

Fermentation broths containing AA can be produced from the *Candida tropicalis* (Castellani) Berkhout, anamorph strain OH23 having ATCC accession number 24887 by culture at 32°C in a liquid medium containing 300 mg of NH₄H2PO₄, 200 mg of KH₂PO₄, 100 mg of K₂HPO₄, 50 mg of MgSO₄·7H₂O, 1 µg of biotin, 0.1% (w/v) yeast extract and about 1% (v/v) *n-*hexadecane in 100 ml of distilled water. Other culture media such as YM broth containing *n-*hexadecane may also be used. The procedure for producing fermentation broths containing AA from media containing *n*-hexadecane by culturing *Candid tropicalis* (Castellani) Berkhout, anamorph strain OH23 having ATCC accession number 24887 is also described in Okuhura et al., 35 Agr. Biol. Chem. 1376 (1971) the subject matter of which is incorporated herein by reference.

Fermentation broths containing AA can also be produced from *E. coli* strain AB2834/pKD136/pKD8.243A/pKD8.292 having ATCC accession number 69875. This can be done as follows. One liter ofLB medium (in 4 L Erlenmeyer shake flask) containing IPTG (0.2 mM), ampicillin (0.05 g), chloramphenicol (0.02 g) and spectinomycin (0.05 g) can be inoculated with 10 mL of an overnight culture of *E. coli* strain AB2834/pKD136/pKD8.243A/pKD8.292 cells grown at 250 rpm for 10 h at 37°C. The cells can be harvested, resuspended in 1 L of M9 minimal medium containing 56 mM D-glucose, shikimic acid (0.04 g), IPTG (0.2 mM), ampicillin (0.05 g), chloramphenicol (0.02 g) and spectinomycin (0.05 g). The cultures can then be returned to 37°C incubation. After resuspension in minimal medium the pH of the culture can be closely monitored, particularly over the initial 12 h. When the culture reaches a pH of 6.5, 5N NaOH or an appropriate amount of another base such as ammonium hydroxide can be added to adjust the pH back to approximately 6.8. Over the 48 h accumulation period, the culture should not allowed to fall below pH 6.3. After 24 h in the medium 12 mM *cis, cis*-muconate and 1 mM protocatechuate may be detected in the culture supernatant along with 23 mM D-glucose. After 48 h in the medium *E. coli* strain AB2834/pKD136/pKD8.243A/pKD8.292 cells can essentially replace the 56 mM D-glucose in the medium with 17 mM *cis, cis*-muconate.

The reduction of microbially synthesized *cis, cis*-muconate AA to produce a fermentation broth containing AA can then proceed as follows. Fifty milligrams of platinum on carbon (10%) can be added to 6 mL of a cell-free culture supernatant from the fermentation containing about 17.2 mM *cis, cis*-muconate. This sample can then be hydrogenated at 50 psi hydrogen pressure for 3 h at room temperature to produce a fermentation broth containing AA. The fermentation broth produced in this fashion may contain, for example, about 15.1 mM AA. The procedure for producing fermentation broths containing AA by culturing *E. coli* strain AB2834/pKD136/pKD8.243A/pKD8.292 cells by culture in a growth medium comprising D-glucose is also described in Draths & Frost, 116 J. Am. Chem. Soc. 399 (1994); Draths and Frost, 18 Biotechnol. Prog. 201 (2002); US 5,487,987 and US 5,616,496 the subject matter of which is incorporated herein by reference..

Fermentation broths containing AA can also be produced from the *E. coli* cosmid clones designated 5B12, 5F5, 8F6 and 14D7 comprising a vector expressing the cyclohexanone monoxygenase SEQ ID NO: 2 encoded by SEQ ID NO: 1 from *Acinetobacter* strain SE19 by culturing these clones in M9 minimal medium supplemented with 0.4% glucose as the carbon source. Cells can be grown at 30°C with shaking for 2 h and the addition of 330 ppm of cyclohexanol to the medium. This can be followed by further incubation at 30°C for an additional period of time such as, for example, 2 h, 4 h or 20 h or other time intervals. The procedure for producing fermentation broths containing AA by culturing the *E. coli* cosmid clones designated 5B12, 5F5, 8F6 and 14D7 comprising a vector expressing the cyclohexanone monoxygenase encoded by SEQ ID NO: 1 from *Acinetobacter* strain SE19 in a growth medium comprising D-glucose and clyclohexanol is also described in US 6,794,165, the subject matter of which is incorporated herein by reference.

Fermentation broths containing AA can also be produced with the Verdezyne Yeast strain available from Verdezyne, Inc. (Carslbad, CA, USA) which was reported on February 8, 2010 to produce AA when cultured in a medium (e.g., SD medium) comprising alkanes or other carbon sources such as sugars and plant-based oils.

Fermentation broths containing AA can also be produced from *E. coli* or other microorganisms transformed with nucleic acids encoding succinyl-CoA:acetyl-CoA acyl transferase; 3-hydroxyacyl-CoA dehydrogenase; 3-hydroxyadipyl-CoA dehydratase; 5-carboxy-2-pentenoyl-CoA reductase; adipyl-CoA synthetase, phosphotransadipylase/adipate kinase, adipyl-CoA transferase or adipyl-CoA hydrolase. Fermentation broths containing AA can further be produced from *E. coli* or other microorganisms transformed with nucleic acids encoding succinyl-CoA:acetyl-CoA acyl transferase; 3-oxoadipyl-CoA transferase; 3-oxoadipate reductase; 3-hydroxyadipate dehydratase; and 2-enoate reductase. Fermentation broths containing AA can also be produced from *E. coli* or other microorganisms transformed with nucleic acids encoding alpha-ketoadipyl-CoA synthetase, phosphotransketoadipylase/alpha-ketoadipate kinase or alpha-ketoadipyl-CoA:acetl-CoA tranferase; 2-hydroxyadipyl-CoA dehydrogenase; 2-hydroxyadipyl-CoA dehydratase; 5-carboxy-2-penteoyl-CoA reductase; and adipyl-CoA synthetase, phosphotransadipylase/adipate kinase, adipyl-CoA:acetyl-CoA transferase or adipyl-CoA hydrolase. Fermentation broths containing AA can still further be produced from *E. coli* or other microorganisms transformed with nucleic acids encoding 2-hydroxyadipate dehydrogenase; 2-hydroxyadipyl-CoA synthetase, phosphotranshydroxyadipylase/2-hydroxyadipate kinase or 2-hydroxyadipyl-CoA:acetyl-CoA transferase; 2-hydroxyadipyl-CoA dehydratase; 5-carboxy-2-pentenoyl-CoA reductase; and adipyl-CoA synthetase, phosphotransadipylase/adipate kinase, adipyl-CoA:acetyl-CoA transferase or adipyl-CoA hydrolase.

Fermentations with *E. coli* or other microorganisms transformed with nucleic acids encoding these enzymes may be performed using a variety of different carbon sources under standard conditions in standard culture mediums (e.g., M9 minimal medium) and appropriate antibiotic or nutritional supplements necessary to maintain the transformed phenotype. The procedure for producing fermentation broths containing AA by culturing *E. coli* or other microorganisms transformed with nucleic acids encoding these enzymes, appropriate growth mediums and carbon sources are also described in US 2009/0305364, the subject matter of which is incorporated herein by reference.

Procedures for producing fermentation broths containing dicarboxylic acids such as AA by culturing *Saccharomyces cerevisiae* and other strains, microorganism strains, appropriate growth mediums and carbon sources are also described in WO 2010/003728, the subject matter of which is incorporated herein by reference.

A fermentable carbon source (e.g., carbohydrates and sugars), optionally a source of nitrogen and complex nutrients (e.g., corn steep liquor), additional media components such as vitamins, salts and other materials that can improve cellular growth and/or product formation, and water may be fed to the growth vessel 12 for growth and sustenance of the microbial culture. Typically, the microbial culture is grown under aerobic conditions provided by sparging an oxygen-rich gas (e.g., air or the like). Typically, an acid (e.g., sulphuric acid or the like) and ammonium hydroxide are provided for pH control during the growth of the microbial culture.

In one example (not shown), the aerobic conditions in growth vessel 12 (provided by sparging an oxygen-rich gas) are switched to anaerobic conditions by changing the oxygen-rich gas to an oxygen-deficient gas (e.g., CO₂ or the like). The anaerobic environment may trigger bioconversion of the fermentable carbon source to AA *in situ* in growth vessel 12. Ammonium hydroxide is provided for pH control during bioconversion of the fermentable carbon source to AA. The AA that is produced is at least partially if not totally neutralized to DAA due to the presence of the ammonium hydroxide, leading to the production of a broth comprising DAA. The addition of CO₂ may provides an additional source of carbon for the production of AA.

In another example, the contents of growth vessel 12 may be transferred via stream 14 to a separate bioconversion vessel 16 for bioconversion of a carbohydrate source to AA. An oxygen-deficient gas (e.g., CO₂ or the like) is sparged in bioconversion vessel 16 to provide anaerobic conditions that trigger production of AA. Ammonium hydroxide is provided for pH control during bioconversion of the carbohydrate source to AA. Due to the presence of the ammonium hydroxide, the AA produced is at least partially neutralized to DAA, leading to production of a broth that comprises DAA. The addition of CO₂ may provide an additional source of carbon for production of AA.

In yet another example, the bioconversion may be conducted at relatively low pH (e.g., 3 - 6). Abase (ammonium hydroxide or ammonia) may be provided for pH control during bioconversion of the carbohydrate source to AA. Depending of the desired pH, due to the presence or lack of the ammonium hydroxide, either AA is produced or the AA produced is at least partially neutralized to MAA, DAA or a mixture comprising AA, MAA and/or DAA. Thus, the AA produced during bioconversion can be subsequently neutralized, optionally in an additional step, by providing either ammonia or ammonium hydroxide leading to a broth comprising DAA. As a consequence, a "DAA-containing fermentation broth" generally means that the fermentation broth comprises DAA and possibly any number of other components such as MAA and/or AA, whether added and/or produced by bioconversion or otherwise. Similarly, a "MAA-containing fermentation broth" generally means that the fermentation broth comprises MAA and possibly any number of other components such as DAA and/or AA, whether added and/or produced by bioconversion or otherwise.

The broth resulting from the bioconversion of the fermentable carbon source (in either growth vessel 12 or bioconversion vessel 16, depending on where the bioconversion takes place), typically contains insoluble solids such as cellular biomass and other suspended material, which are transferred via stream 18 to clarification apparatus 20 before distillation. Removal of insoluble solids clarifies the broth. This reduces or prevents fouling of subsequent distillation equipment. The insoluble solids can be removed by any one of several solid-liquid separation techniques, alone or in combination, including but not limited to centrifugation and filtration (including, but not limited to ultra-filtration, micro-filtration or depth filtration). The choice of filtration can be made using techniques known in the art. Soluble inorganic compounds can be removed by any number of known methods such as, but not limited to, ion-exchange, physical adsorption and the like.

An example of centrifugation is a continuous disc stack centrifuge. It may be useful to add a polishing filtration step following centrifugation such as dead-end or cross-flow filtration that may include the use of a filter aid such as diatomaceous earth or the like, or more preferably ultra-filtration or micro-filtration. The ultra-filtration or micro-filtration membrane can be ceramic or polymeric, for example. One example of a polymeric membrane is SelRO MPS-U20P (pH stable ultra-filtration membrane) manufactured by Koch Membrane Systems (850 Main Street, Wilmington, MA, USA). This is a commercially available polyethersulfone membrane with a 25,000 Dalton molecular weight cut-off which typically operates at pressures of 0.35 to 1.38 MPa (maximum pressure of 1.55 MPa) and at temperatures up to 50°C. Alternatively, a filtration step may be employed alone using ultra-filtration or micro-filtration.

The resulting clarified DAA-containing broth or MAA-containing broth, substantially free of the microbial culture and other solids, is transferred via stream 22 to distillation apparatus 24.

The clarified distillation broth should contain DAA in an amount that is at least a majority of, preferably at least about 70 wt%, more preferably 80 wt% and most preferably at least about 90 wt% of all the diammonium dicarboxylate salts in the broth. The concentration of DAA and/or MAA as a weight percent (wt%) of the total dicarboxylic acid salts in the fermentation broth can be determined by high pressure liquid chromatography (HPLC) or other known means.

Water and ammonia are removed from distillation apparatus 24 as an overhead, and at least a portion is optionally recycled via stream 26 to bioconversion vessel 16 (or growth vessel 12 operated in the anaerobic mode).

The specific distillation temperature and pressure are not critical as long as the distillation is carried out in a way that ensures that the distillation overhead contains water and ammonia, and the distillation bottoms comprises at least some AA and at least about 20 wt% water. A more preferred amount of water is at least about 30 wt% and an even more preferred amount is at least about 40 wt%. The rate of ammonia removal from the distillation step increases with increasing temperature and also can be increased by injecting steam (not shown) during distillation. The rate of ammonia removal during distillation may also be increased by conducting distillation under a vacuum or by sparging the distillation apparatus with a nonreactive gas such as air, nitrogen or the like.

Removal of water during the distillation step can be enhanced by the use of an organic azeotroping agent such as toluene, xylene, methylcyclohexane, methyl isobutyl ketone, cyclohexane, heptane or the like, provided that the bottoms contains at least about 20 wt% water. If the distillation is carried out in the presence of an organic agent capable of forming an azeotrope consisting of the water and the agent, distillation produces a biphasic bottoms that comprises an aqueous phase and an organic phase, in which case the aqueous phase can be separated from the organic phase, and the aqueous phase used as the distillation bottoms. By-products such as adipimide and adipamide are substantially avoided provided the water level in the bottoms is maintained at a level of at least about 30 wt%.

A preferred temperature for the distillation step is in the range of about 50 to about 300°C, depending on the pressure. A more preferred temperature range is about 150 to about 240°C, depending on the pressure. A distillation temperature of about 170 to about 230°C is preferred. "Distillation temperature" refers to the temperature of the bottoms (for batch distillations this may be the temperature at the time when the last desired amount of overhead is taken).

Adding a water miscible organic solvent or an ammonia separating solvent facilitates deammoniation over a variety of distillation temperatures and pressures as discussed above. Such solvents include aprotic, bipolar, oxygen-containing solvents that may be able to form passive hydrogen bonds. Examples include, but are not limited to, diglyme, triglyme, tetraglyme, sulfoxides such as dimethylsulfoxide (DMSO), amides such as dimethylformamide (DMF) and dimethylacetamide, sulfones such as dimethylsulfone, gamma-butyrolactone (GBL), sulfolane, polyethyleneglycol (PEG), butoxytriglycol, N-methylpyrolidone (NMP), ethers such as dioxane, methyl ethyl ketone (MEK) and the like. Such solvents aid in the removal of ammonia from the DAA or MAA in the clarified broth. Regardless of the distillation technique, it is important that the distillation be carried out in a way that ensures that at least some MAA and at least about 20 wt% water remain in the bottoms and even more advantageously at least about 30 wt%. The distillation can be performed at atmospheric, sub-atmospheric or superatmospheric pressures.

Under other conditions such as when the distillation is conducted in the absence of an azeotropic agent or ammonia separating solvent, the distillation is conducted at super atmospheric pressure at a temperature of greater than 100°C to about 300°C to form an overhead that comprises water and ammonia and a liquid bottoms that comprises AA and at least about 20 wt% water. Super atmospheric pressure typically falls within a range of greater than ambient atmosphere up to and including about 25 atmospheres. Advantageously the amount of water is at least about 30 wt%.

The distillation can be a one-stage flash, a multistage distillation (i.e., a multistage column distillation) or the like. The one-stage flash can be conducted in any type of flasher (e.g., a wiped film evaporator, thin film evaporator, thermosiphon flasher, forced circulation flasher and the like). The multistages of the distillation columns can be achieved by using trays, packing or the like. The packing can be random packing (e.g., Raschig rings, Pall rings, Ber1 saddles and the like) or structured packing (e.g., Koch-Sulzer packing, Intalox packing, Mellapak and the like). The trays can be of any design (e.g., sieve trays, valve trays, bubble-cap trays and the like). The distillation can be performed with any number of theoretical stages.

If the distillation apparatus is a column, the configuration is not particularly critical, and the column can be designed using well known criteria. The column can be operated in either stripping mode, rectifying mode or fractionation mode. Distillation can be conducted in either batch, semi-continuous or continuous mode. In the continuous mode, the broth is fed continuously into the distillation apparatus, and the overhead and bottoms are continuously removed from the apparatus as they are formed. The distillate from distillation is an ammonia/water solution, and the distillation bottoms is a liquid, aqueous solution of MAA and AA, which may also contain other fermentation by-product salts (i.e., ammonium acetate, ammonium formate, ammonium lactate and the like) and color bodies.

The distillation bottoms can be transferred via stream 28 to cooling apparatus 30 and cooled by conventional techniques. Cooling technique is not critical. A heat exchanger (with heat recovery) can be used. A flash vaporization cooler can be used to cool the bottoms down to about 15°C. Cooling to 15°C typically employs a refrigerated coolant such as, for example, glycol solution or, less preferably, brine. A concentration step can be included prior to cooling to help increase product yield. Further, both concentration and cooling can be combined using known methods such as vacuum evaporation and heat removal using integrated cooling jackets and/or external heat exchangers.

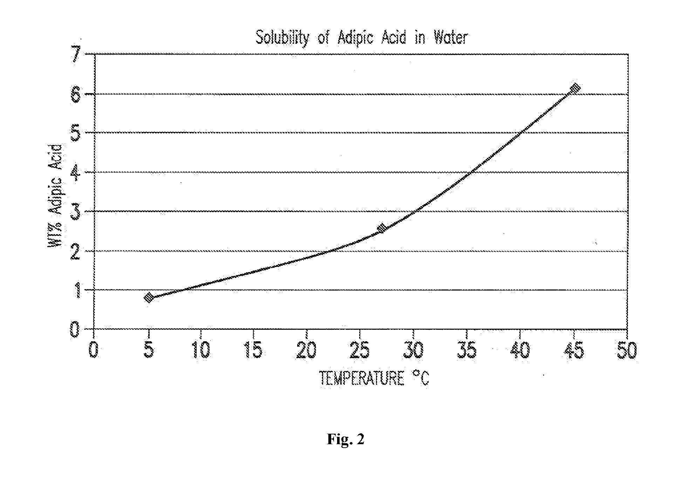

We found that the presence of some MAA in the liquid bottoms facilitates cooling-induced separation of the bottoms into a liquid portion in contact with a solid portion that at least "consists essentially" of AA (meaning that the solid portion is at least substantially pure crystalline AA) by reducing the solubility of AA in the liquid, aqueous, MAA-containing bottoms. Fig. 2 illustrates the solubility of AA in water. We discovered, therefore, that AA can be more completely crystallized out of an aqueous solution if some MAA is also present in that solution. A preferred concentration of MAA in such a solution is about 20 wt% or higher. This phenomenon allows crystallization of AA (i.e., formation of the solid portion of the distillation bottoms) at temperatures higher than those that would be required in the absence of MAA.

The distillation bottoms is fed via stream 32 to separator 34 for separation of the solid portion from the liquid portion. Separation can be accomplished via pressure filtration (e.g., using Nutsche or Rosenmond type pressure filters), centrifugation and the like. The resulting solid product can be recovered as product 36 and dried, if desired, by standard methods.

After separation, it may be desirable to treat the solid portion to ensure that no liquid portion remains on the surface(s) of the solid portion. One way to minimize the amount of liquid portion that remains on the surface of the solid portion is to wash the separated solid portion with water and dry the resulting washed solid portion (not shown). A convenient way to wash the solid portion is to use a so-called "basket centrifuge" (not shown). Suitable basket centrifuges are available from The Western States Machine Company (Hamilton, OH, USA).

The liquid portion of the distillation bottoms 34 (i.e., the mother liquor) may contain remaining dissolved AA, any unconverted MAA, any fermentation by-products such as ammonium acetate, lactate, or formate, and other minor impurities. This liquid portion can be fed via stream 38 to a downstream apparatus 40. In one instance, apparatus 40 may be a means for making a de-icer by treating in the mixture with an appropriate amount of potassium hydroxide, for example, to convert the ammonium salts to potassium salts. Ammonia generated in this reaction can be recovered for reuse in the bioconversion vessel 16 (or growth vessel 12 operating in the anaerobic mode). The resulting mixture of potassium salts is valuable as a de-icer and anti-icer.

The mother liquor from the solids separation step 34 can be recycled (or partially recycled) to distillation apparatus 24 via stream 42 to further enhance recovery of AA, as well as further convert MAA to AA.

The solid portion of the cooling-induced crystallization is substantially pure AA and is, therefore, useful for the known utilities of AA.

HPLC can be used to detect the presence of nitrogen-containing impurities such as adipamide and adipimide. The purity of AA can be determined by elemental carbon and nitrogen analysis. An ammonia electrode can be used to determine a crude approximation of AA purity.

Depending on the circumstances and various operating inputs, there are instances when the fermentation broth may be a clarified MAA-containing fermentation broth or a clarified AA-containing fermentation broth. In those circumstances, it can be advantageous to add MAA, DAA and/or AA and, optionally, ammonia and/or ammonium hydroxide to those fermentation broths to facilitate the production of substantially pure AA. For example, the operating pH of the fermentation broth may be oriented such that the broth is a MAA-containing broth or a AA-containing broth. MAA, DAA, AA, ammonia and/or ammonium hydroxide may be added to those broths to attain a broth pH preferably less than 6 to facilitate production of the above-mentioned substantially pure AA. In one particular form, it is especially advantageous to recycle AA, MAA and water from the liquid bottoms resulting from the distillation step 24 into the fermentation broth and/or clarified fermentation broth. In referring to the MAA-containing broth, such broth generally means that the fermentation broth comprises MAA and possibly any number of other components such as DAA and/or AA, whether added and/or produced by bioconversion or otherwise.

### Examples

Our processes are illustrated by the following non-limiting representative examples.

The use of a synthetic DAA solution is believed to be a good model for the behavior of an actual broth in our processes because of the solubility of the typical fermentation by-products found in actual broth. The major by-products produced during fermentation are ammonium acetate, ammonium lactate and ammonium formate. Ammonium acetate, ammonium lactate and ammonium formate are significantly more soluble in water than AA, and each is typically present in the broth at less than 10% of the DAA concentration. In addition, even when the acids (acetic, formic and lactic acids) were formed during the distillation step, they are miscible with water and will not crystallize from water. This means that the AA reaches saturation and crystallizes from solution (i.e., forming the solid portion), leaving the acid impurities dissolved in the mother liquor (i.e., the liquid portion).

### Example 1

This example shows the conversion of DAA to MAA.

A 1-L round bottom flask was charged with 800g of a synthetic 4.5% DAA solution. The flask was fitted with a five tray Oldershaw section which was capped with a distillation head. The distillate was collected in an ice cooled receiver. The contents of the flask were heated with a heating mantel and stirred with a magnetic stirrer. Distillation was started and 719.7g of distillate collected. Titration of the distillate revealed it was a 0.29% ammonia solution (i.e. an about 61% conversion of DAA to MAA). The hot residue (76g) was discharged from the flask and placed in an Erlenmeyer flask and slowly cooled to room temperature while stirring over a weekend. The contents were then cooled to 15°C for 60 minutes and then cooled to 10°C for 60 minutes and finally 5°C for 60 minutes while stirring. The solids were filtered and dried in a vacuum oven for 2 hour at 75° yielding 16.2g. Analysis of the solids for ammonia content by ammonia electrode indicated it was approximately a 1:1 molar ratio of ammonia and AA.

### Example 2

This example shows the conversion of MAA to AA.

A 300 mL Parr autoclave was charged with 80g of synthetic MAA and 124g of water. The autoclave was sealed and the contents stirred and heated to about 200°C (autogenic pressure was about 203 psig). Once the contents reached temperature, water was then fed to the autoclave at a rate of about 2 g/min and vapor was removed from the autoclave at a rate of about 2g/min by means of a back pressure regulator. The vapor exiting the autoclave was condensed and collected in a receiver. The autoclave was run under these conditions until a total of 1210g of water had been fed and a total of 1185g of distillate collected. The contents of the autoclave (209g) were partially cooled and discharged from the reactor. The slurry was allowed to stand stirring at room temperature over night in an Erlenmeyer flask. The slurry was then filtered and the solids rinsed with 25g of water. The moist solids were dried in a vacuum oven at 75°C for 1 hr yielding 59g of AA product. Analysis via an ammonium ion electrode revealed 0.015 mmole ammonium ion/g of solid. The melting point of the recovered solid was 151 to 154°C.

### Example 3

This example shows the conversion of DAA to MAA in the presence of a solvent.

A beaker was charged with 36.8g of distilled water and 19.7g of concentrated ammonium hydroxide. Then 23.5g of adipic acid was slowly added. The mixture was stirred forming a clear solution which was then placed in a 500 mL round bottom flask which contained a stir bar. Triglyme (80g) was then added to the flask. The flask was then fitted with a 5 tray 1" Oldershaw column section which was topped with a distillation head. The distillation head was fitted with an ice bath cooled receiver. The distillation flask was also fitted with an addition funnel which contained 150g of distilled water. The contents were then stirred and heated with a heating mantel. When distillate began to come over the water in the addition funnel was added dropwise to the flask at the same rate as the distillate take-off. The distillation was stopped when all of the water in the addition funnel had been added. A total of 158g of distillate had been collected. Titration of the distillate revealed a 1.6% ammonia content. This is equivalent to 46% of the charged ammonia. In other words the residue is a 91/9 mixture of monoammonium adipate/diammonium adipate. After cooling to room temperature, the residue was place in a 250 mL Erlenmeyer flask and slowly cooled to 5°C while stirring. The slurry was filtered and the wet crystals were then dried in a vacuum oven for 2 hours yielding 5.5g of solids. Analysis of the solids indicated essentially a one to one ratio of ammonium ion to adipate ion (i.e. monoammonium adipate).

### Example 4

This example shows the conversion of MAA to AA in the presence of a solvent.

A beaker was charged with 46.7g of distilled water and 9.9g of concentrated ammonium hydroxide. Then 23.5g of adipic acid was slowly added. The mixture was stirred forming a clear solution which was then placed in a 500 mL round bottom flask which contained a stir bar. Triglyme (80g) was then added to the flask. The flask was then fitted with a 5 tray 1" Oldershaw column section which was topped with a distillation head. The distillation head was fitted with an ice bath cooled receiver. The distillation flask was also fitted with an addition funnel which contained 1800g of distilled water. The contents were then stirred and heated with a heating mantel. When distillate began to come over the water in the addition funnel was added dropwise to the flask at the same rate as the distillate take-off. The distillation was stopped when all of the water in the addition funnel had been added. A total of 1806.2g of distillate had been collected. Titration of the distillate revealed a 0.11% ammonia content. This is equivalent to 72% of the charged ammonia. In other words the residue is a 72/28 mixture of adipic acid/monoammonium adipate. The residue was then placed in an Erlenmeyer flask and cooled to 0° C while stirring and allowed to stand for 1 hr. The slurry was filtered yielding 18.8g of a wet cake and 114.3g of mother liquor. The solids were then dried under vacuum at 80°C for 2 hrs yielding 13.5g of solids. The solids were then dissolved in 114g of hot water and then cooled to 5°C and held stirring for 45 minutes. The slurry was filtered yielding 13.5g of wet solids and 109.2g of mother liquor. The solids were dried under vacuum at 80°C for 2 hrs yielding 11.7g of dried solids. Analysis of the solids revealed an ammonium ion content of 0.0117 mmol/g (i.e. essentially pure adipic acid).

Although our processes have been described in connection with specific steps and forms thereof, it will be appreciated that a wide variety of equivalents may be substituted for the specified elements and steps described herein without departing from the spirit and scope of this disclosure as described in the appended claims.

## Claims

1. A process for making AA from a clarified MAA or DAA-containing fermentation broth comprising:
(a) distilling the broth under super atmospheric pressure at a temperature of >100°C to about 300°C to form an overhead that comprises water and ammonia, and a liquid bottoms that comprises AA, and at least about 20 wt% water;
(b) cooling and/or evaporating the bottoms to attain a temperature and composition sufficient to cause the bottoms to separate into a liquid portion and a solid portion that is substantially pure AA; and
(c) separating the solid portion from the liquid portion.

2. A process for making AA from a clarified MAA or DAA-containing fermentation broth comprising:
(a) adding an ammonia separating and/or water azeotroping solvent to the broth;
(b) distilling the broth at a temperature and pressure sufficient to form an overhead that comprises water and ammonia, and a liquid bottoms that comprises AA, and at least about 20 wt% water;
(c) cooling and/or evaporating the bottoms to attain a temperature and composition sufficient to cause the bottoms to separate into a liquid portion and a solid portion that is substantially pure AA; and
(d) separating the solid portion from the liquid portion.

3. The process of any of the preceding claims, wherein the fermentation broth is obtained by fermenting a carbon source in the presence of a microorganism selected from the group consisting of *Candida tropicalis* (Castellani) Berkhout, anamorph strain OH23 having ATCC accession number 24887; *E. coli* strain AB2834/pKD136/pKD8.243A/pKD8.292 having ATCC accession number 69875; *E. coli* cosmid clone 5B12 comprising a vector expressing the cyclohexanone monoxygenase encoded by SEQ ID NO: 1, *E. coli* cosmid clone 5F5 comprising a vector expressing the cyclohexanone monoxygenase encoded by SEQ ID NO: 1; *E. coli* cosmid clone 8F6 comprising a vector expressing the cyclohexanone monoxygenase encoded by SEQ ID NO: 1; *E. coli* cosmid clone 14D7 comprising a vector expressing the cyclohexanone monoxygenase encoded by SEQ ID NO: 1; and Verdezyne Yeast.

4. The process of any of the preceding claims, wherein the liquid bottoms contains at least some MAA and the solid portion is substantially free of adipamic acid, adipamide and adipimide.

5. The process of any of the preceding claims, further comprising recycling the AA, water, and the solvent and, optionally, MAA from the liquid bottoms to the clarified broth.

6. The process of any of the preceding claims, wherein distilling the broth is carried out in the presence of an ammonia separating solvent which is at least one selected from the group consisting of diglyme, triglyme, tetraglyme, sulfoxides, amides, sulfones, polyethyleneglycol (PEG), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

7. The process of any of the preceding claims, further comprising recycling the AA and water and optionally MAA from the liquid bottoms to the clarified broth.

8. The process of any of the preceding claims, further comprising removing water from the liquid bottoms to increase concentration of AA in the liquid bottoms.


SEQUENCE LISTING

<110> FRUCHEY, OLAN S.
      MANZER, LEO E.
      DUNUWILA, DILUM
      KEEN, BRIAN T.
      ALBIN, BROOKE A.
      CLINTON, NYE A.
      DOMBEK, BERNARD D.

<120> PROCESSES FOR PRODUCING ADIPIC ACID FROM FERMENTATION BROTHS
      CONTAINING DIAMMONIUM ADIPATE

<140> 13/087,001
<141> 2011-04-14

<150> 61/329,800
<151> 2010-04-30

<160> 2

<170> PatentIn version 3.5

<210> 1
<211> 1644
<212> DNA
<213> Acinetobacter sp.

<400> 1
atggagatta tcatgtcaca aaaaatggat tttgatgcta tcgtgattgg tggtggtttt      60

ggcggacttt atgcagtcaa aaaattaaga gacgagctcg aacttaaggt tcaggctttt     120

gataaagcca cggatgtcgc aggtacttgg tactggaacc gttacccagg tgcattgtcg     180

gatacagaaa cccacctcta ctgctattct tgggataaag aattactaca atcgctagaa     240

atcaagaaaa aatatgtgca aggccctgat gtacgcaagt atttacagca agtggctgaa     300

aagcatgatt taaagaagag ctatcaattc aataccgcgg ttcaatcggc tcattacaac     360

gaagcagatg ccttgtggga agtcaccact gaatatggtg ataagtacac ggcgcgtttc     420

ctcatcactg ctttaggctt attgtctgcg cctaacttgc caaacatcaa aggcattaat     480

cagtttaaag gtgagctgca tcataccagc cgctggccag atgacgtaag ttttgaaggt     540

aaacgtgtcg gcgtgattgg tacgggttcc accggtgttc aggttattac ggctgtggca     600

cctctggcta aacacctcac tgtcttccag cgttctgcac aatacagcgt tccaattggc     660

aatgatccac tgtctgaaga agatgttaaa aagatcaaag acaattatga caaaatttgg     720

gatggtgtat ggaattcagc ccttgccttt ggcctgaatg aaagcacagt gccagcaatg     780

agcgtatcag ctgaagaacg caaggcagtt tttgaaaagg catggcaaac aggtggcggt     840

ttccgtttca tgtttgaaac tttcggtgat attgccacca atatggaagc caatatcgaa     900

gcgcaaaatt tcattaaggg taaaattgct gaaatcgtca aagatccagc cattgcacag     960

aagcttatgc cacaggattt gtatgcaaaa cgtccgttgt gtgacagtgg ttactacaac    1020

acctttaacc gtgacaatgt ccgtttagaa gatgtgaaag ccaatccgat tgttgaaatt 1080

accgaaaacg gtgtgaaact cgaaaatggc gatttcgttg aattagacat gctgatatgt 1140

gccacaggtt ttgatgccgt cgatggcaac tatgtgcgca tggacattca aggtaaaaac 1200

ggcttggcca tgaaagacta ctggaaagaa ggtccgtcga gctatatggg tgtcaccgta 1260

aataactatc caaacatgtt catggtgctt ggaccgaatg gcccgtttac caacctgccg 1320

ccatcaattg aatcacaggt ggaatggatc agtgatacca ttcaatacac ggttgaaaac 1380

aatgttgaat ccattgaagc gacaaaagaa gcggaagaac aatggactca aacttgcgcc 1440

aatattgcgg aaatgacctt attccctaaa gcgcaatcct ggatttttgg tgcgaatatc 1500

ccgggcaaga aaaacacggt ttacttctat ctcggtggtt taaaagaata tcgcagtgcg 1560

ctagccaact gcaaaaacca tgcctatgaa ggttttgata ttcaattaca acgttcagat 1620

atcaagcaac ctgccaatgc ctaa 1644

<210> 2
<211> 547
<212> PRT
<213> Acinetobacter sp.

<400> 2
Met Glu Ile Ile Met Ser Gln Lys Met Asp Phe Asp Ala Ile Val Ile
1               5                   10                  15

Gly Gly Gly Phe Gly Gly Leu Tyr Ala Val Lys Lys Leu Arg Asp Glu
            20                  25                  30

Leu Glu Leu Lys Val Gln Ala Phe Asp Lys Ala Thr Asp Val Ala Gly
        35                  40                  45

Thr Trp Tyr Trp Asn Arg Tyr Pro Gly Ala Leu Ser Asp Thr Glu Thr
    50                  55                  60

His Leu Tyr Cys Tyr Ser Trp Asp Lys Glu Leu Leu Gln Ser Leu Glu
65                  70                  75                  80

Ile Lys Lys Lys Tyr Val Gln Gly Pro Asp Val Arg Lys Tyr Leu Gln
                85                  90                  95

Gln Val Ala Glu Lys His Asp Leu Lys Lys Ser Tyr Gln Phe Asn Thr
            100                 105                 110

Ala Val Gln Ser Ala His Tyr Asn Glu Ala Asp Ala Leu Trp Glu Val
        115                 120                 125

Thr Thr Glu Tyr Gly Asp Lys Tyr Thr Ala Arg Phe Leu Ile Thr Ala
    130                 135                 140

Leu Gly Leu Leu Ser Ala Pro Asn Leu Pro Asn Ile Lys Gly Ile Asn
145                 150                 155                 160

Gln Phe Lys Gly Glu Leu His His Thr Ser Arg Trp Pro Asp Asp Val
                165                 170                 175

Ser Phe Glu Gly Lys Arg Val Gly Val Ile Gly Thr Gly Ser Thr Gly
            180                 185                 190

Val Gln Val Ile Thr Ala Val Ala Pro Leu Ala Lys His Leu Thr Val
        195                 200                 205

Phe Gln Arg Ser Ala Gln Tyr Ser Val Pro Ile Gly Asn Asp Pro Leu
    210                 215                 220

Ser Glu Glu Asp Val Lys Lys Ile Lys Asp Asn Tyr Asp Lys Ile Trp
225                 230                 235                 240

Asp Gly Val Trp Asn Ser Ala Leu Ala Phe Gly Leu Asn Glu Ser Thr
                245                 250                 255

Val Pro Ala Met Ser Val Ser Ala Glu Glu Arg Lys Ala Val Phe Glu
            260                 265                 270

Lys Ala Trp Gln Thr Gly Gly Gly Phe Arg Phe Met Phe Glu Thr Phe
        275                 280                 285

Gly Asp Ile Ala Thr Asn Met Glu Ala Asn Ile Glu Ala Gln Asn Phe
    290                 295                 300

Ile Lys Gly Lys Ile Ala Glu Ile Val Lys Asp Pro Ala Ile Ala Gln
305                 310                 315                 320

Lys Leu Met Pro Gln Asp Leu Tyr Ala Lys Arg Pro Leu Cys Asp Ser
                325                 330                 335

Gly Tyr Tyr Asn Thr Phe Asn Arg Asp Asn Val Arg Leu Glu Asp Val
            340                 345                 350

Lys Ala Asn Pro Ile Val Glu Ile Thr Glu Asn Gly Val Lys Leu Glu
        355                 360                 365

Asn Gly Asp Phe Val Glu Leu Asp Met Leu Ile Cys Ala Thr Gly Phe
    370                 375                 380

Asp Ala Val Asp Gly Asn Tyr Val Arg Met Asp Ile Gln Gly Lys Asn
385                 390                 395                 400

Gly Leu Ala Met Lys Asp Tyr Trp Lys Glu Gly Pro Ser Ser Tyr Met
                405                 410                 415

Gly Val Thr Val Asn Asn Tyr Pro Asn Met Phe Met Val Leu Gly Pro
            420                 425                 430

Asn Gly Pro Phe Thr Asn Leu Pro Pro Ser Ile Glu Ser Gln Val Glu

435                 440                 445

Trp Ile Ser Asp Thr Ile Gln Tyr Thr Val Glu Asn Asn Val Glu Ser
    450                 455                 460

Ile Glu Ala Thr Lys Glu Ala Glu Glu Gln Trp Thr Gln Thr Cys Ala
465                 470                 475                 480

Asn Ile Ala Glu Met Thr Leu Phe Pro Lys Ala Gln Ser Trp Ile Phe
                485                 490                 495

Gly Ala Asn Ile Pro Gly Lys Lys Asn Thr Val Tyr Phe Tyr Leu Gly
            500                 505                 510

Gly Leu Lys Glu Tyr Arg Ser Ala Leu Ala Asn Cys Lys Asn His Ala
        515                 520                 525

Tyr Glu Gly Phe Asp Ile Gln Leu Gln Arg Ser Asp Ile Lys Gln Pro
    530                 535                 540

Ala Asn Ala
545